# EUROPEAN PATENT APPLICATION

(11) **EP 4 613 733 A1**
(43) Date of publication of application: **10.09.2025**
(21) Application number: 23885619.9
(22) Date of filing: 25.10.2023
(51) Int. Cl.: C07C 63/24, C07C 63/26, C07C 63/307, C07C 63/331, C07C 229/62, C07C 323/62, C07D 207/34, C07F 5/00, C07F 5/06, C07F 7/00, C07F 7/28, C07F 15/06

(54) **METAL ORGANIC FRAMEWORK**

(30) Priority: 31.10.2022 JP 2022174075; 31.10.2022 JP 2022174076
(71) Applicant: Kyoto University, Kyoto-shi, Kyoto 606-8501 (JP); Sumitomo Chemical Company, Limited, Tokyo 103-6020 (JP)
(72) Inventor: KITAGAWA, Susumu, Kyoto-shi, Kyoto 606-8501 (JP); HIGUCHI, Masakazu, Kyoto-shi, Kyoto 606-8501 (JP); OTAKE, Kenichi, Kyoto-shi, Kyoto 606-8501 (JP); KIKUCHI, Yuta, Ichihara-shi, Chiba 299-0195 (JP)
(74) Representative: Vossius & Partner Patentanwälte Rechtsanwälte mbB
(86) International application number: PCT/JP2023/038534
(87) International publication number: WO 2024/095862

(57) **Abstract**

An object of the present invention is to provide a MOF having excellent desorption performance. The present invention is a metal organic framework comprising a predetermined organic ligand and metal ion, wherein a porosity is 20 to 75%, and a ratio of a largest pore diameter L to a smallest pore diameter S (the largest pore diameter L/the smallest pore diameter S) determined through the following procedures (a1) to (a3) is 1.06 or more.
(a1) a crystal structure determined through X-ray crystal structure analysis is displayed using a space-filling model, and presence or absence of the through pores is observed under a predetermined condition, to identify a through pore Pₘₐₓ having the largest inscribed circle diameter;
(a2) the longest length is found among inner diameters of the through pore Pₘₐₓ in the predetermined cross section, to specify the largest pore diameter L of the through pore Pₘₐₓ,
(a3) in the cross section in which the largest pore diameter L is determined, the smallest pore diameter S is specified.

## Description

### TECHNICAL FIELD

The present invention relates to a metal organic framework.

### BACKGROUND ART

Metal organic frameworks are also referred to as porous coordination polymers and are a class of materials which form porous structures by coordinate bonds between metal ions and organic ligands, and are expected to adsorb and desorb gas and expected to be applied to catalysts, etc.

For example, Patent Literature 1 discloses a metal organic framework including metal ions, first ligands, second ligands, and optional third ligands. In the metal organic framework: the metal ion is an aluminum ion; the first and second ligands are each an ion of an organic compound that includes a hetero ring having two carboxyl groups; a heteroatom and the angle formed between the carboxyl groups satisfy a predetermined condition; the third ligand is an ion of an organic compound having two carboxyl groups; and the proportion of each of the existing first to third ligands is within a predetermined range.

### CITATION LIST

### PATENT LITERATURE

PTL 1: Japanese Laid-Open Patent Publication No. 2020-176101

### SUMMARY OF THE INVENTION

### PROBLEMS TO BE SOLVED BY THE INVENTION

After the metal organic framework (hereinafter, may be referred to as MOF) has adsorbed substances such as water, carbon dioxide, and hydrogen, the MOF is heated to the specific temperature to allow the adsorbed substances to be desorbed from the MOF, thereby enabling regeneration and use of the MOF. In this case, efficient desorption of the adsorbed substances is desired.

Therefore, an object of the present invention is to provide a MOF having excellent desorption performance.

### SOLUTION TO THE PROBLEMS

The present invention that can achieve the problem is as follows.
[1] A metal organic framework comprising
   an organic ligand and a metal ion, wherein
   a porosity is 20 to 75%,
   a ratio of a largest pore diameter L to a smallest pore diameter S (the largest pore diameter L/the smallest pore diameter S) determined through the following procedures (a1) to (a3) is 1.06 or more,
   the organic ligand includes at least one kind among organic ligands each having a group represented by R(COO⁻)ₙ in which R represents an aromatic hydrocarbon group or pyrrole, and may have a functional group X which is -OH, - NH₂, or -S-S-, and n is 2 or more and 3 or less, and
   the metal ion is an ion of at least one metal selected from the group consisting of Al, Ga, In, Ti, V, Cr, Mn, Fe, Co, Cu, Zr, and Hf,
   (a1) a crystal structure determined through X-ray crystal structure analysis is displayed using a space-filling model, and is displayed so as to include outer perimeters of through pores without any omissions, and presence or absence of the through pores is observed from all directions, to identify a through pore Pₘₐₓ having the largest inscribed circle diameter;
   (a2) in each of a cross section ab, a cross section bc, and a cross section ca of the through pore Pₘₐₓ, the longest length is found among inner diameters of the through pore Pₘₐₓ obtained by cutting the through pore Pₘₐₓ along lines orthogonal to a central axis of the through pore Pₘₐₓ, and a longest one, among the longest length in the cross section ab, the longest length in the cross section bc, and the longest length in the cross section ca, is set as the largest pore diameter L of the through pore Pₘₐₓ,
   the cross section ab being a cut cross section, of the through pore Pₘₐₓ, along a plane parallel to an a-b plane of a unit cell, and being a plane obtained by cutting the through pore Pₘₐₓ where the through pore Pₘₐₓ penetrates,
   if a plurality of the cross sections along which the through pore Pₘₐₓ can be cut where the through pore Pₘₐₓ penetrates are present, the cross section ab being the cross section in which an overlapping portion with the through pore Pₘₐₓ is the largest,
   the cross section bc being a cross section obtained by replacing the cross section ab with the cross section bc and the a-b plane with a b-c plane, and the cross section ca being a cross section obtained by replacing the cross section ab with the cross section ca and the a-b plane with a c-a plane, in identifying of the cross section ab; and
   (a3) in the cross section in which the largest pore diameter L is determined, a smallest length, among inner diameters of the through pore Pₘₐₓ obtained by cutting the through pore Pₘₐₓ along the lines orthogonal to the central axis of the through pore Pₘₐₓ, is set as the smallest pore diameter S.
[2] The metal organic framework according to [1], wherein the ratio of the largest pore diameter L to the smallest pore diameter S (the largest pore diameter L/the smallest pore diameter S) is 4.0 or less.

### ADVANTAGEOUS EFFECTS OF THE INVENTION

According to the present invention, the desorption performance of the MOF can be improved.

### BRIEF DESCRIPTION OF THE DRAWINGS

[FIG. 1] FIG. 1 shows an example in which a crystal structure of a MOF of Example 3 is displayed using a space-filling model and is observed from one direction.
[FIG. 2] FIG. 2 shows an example in which the crystal structure of the MOF of Example 3 is displayed using the space-filling model and is observed from another direction.
[FIG. 3] FIG. 3 shows an example in which the crystal structure of the MOF of Example 3 is displayed using the space-filling model and is observed from still another direction.
[FIG. 4] FIG. 4 shows a largest pore diameter L and a smallest pore diameter S of a through pore Pₘₐₓ.

### DESCRIPTION OF EMBODIMENTS

A MOF of the present invention includes an organic ligand and a metal ion, and has a porosity of 20 to 75%, and the ratio of a largest pore diameter L to a smallest pore diameter S (the largest pore diameter L/the smallest pore diameter S), each of which is determined through predetermined procedures, is 1.06 or more. When the porosity is a predetermined value or more, the adsorbed substances are desorbed easily. When the largest pore diameter L/the smallest pore diameter S is a predetermined value or more, the internal surface area of the MOF tends to be increased, and substances such as water, carbon dioxide, and hydrogen are adsorbed easily.

### 1. Porosity

The porosity of the MOF of the present invention is 20 to 75%, preferably 25% or more, more preferably 30% or more, and further preferably 40% or more, and is preferably 70% or less, more preferably 65% or less, and further preferably 60% or less. **In** addition, from the viewpoint of increasing the amount of desorption, the porosity may be 30% or more and 45% or less.

The porosity can be obtained as follows. As in Examples described below, the MOF of the present invention is measured using X-ray diffraction (XRD) to obtain a crystallographic information file (cif), and the cif is loaded into software named Mercury (The Cambridge Crystallographic Data Centre), whereby the porosity can be obtained using, as an input, a void contact surface having a probe radius of 1.5 Å and an approx. grid spacing of 0.7 Å, based on the obtained information.

### 2. Ratio of largest pore diameter L to smallest pore diameter S (largest pore diameter L/smallest pore diameter S)

The largest pore diameter L/the smallest pore diameter S is 1.06 or more, preferably 1.09 or more, more preferably 1.11 or more, further preferably 1.17 or more, and most preferably 2.0 or more, and is preferably 4.0 or less, more preferably 3.5 or less, and further preferably 3.0 or less.

The largest pore diameter L/the smallest pore diameter S is determined through the following procedures (a1) to (a3).

(a1) A crystal structure determined through X-ray crystal structure analysis is displayed using a space-filling model, and is displayed so as to include the outer perimeters of through pores without any omissions, and the presence or absence of the through pores is observed from all directions, to identify a through pore Pₘₐₓ having the largest inscribed circle diameter.

As in the above-described measurement of the porosity, the X-ray crystal structure analysis is performed using XRD measurement to obtain a cif, and the cif is loaded into the Mercury, whereby a single space-filling model (crystal structure) is obtained.

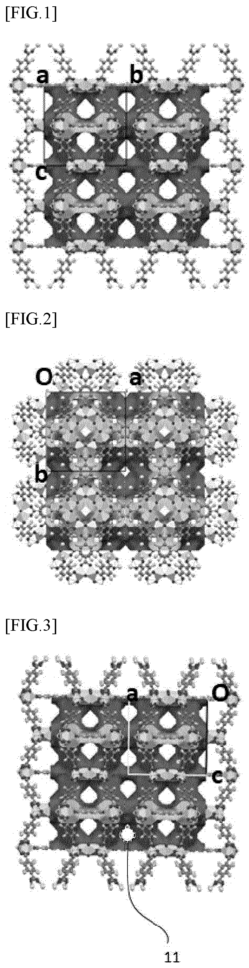

After the space-filling model of the crystal structure has been obtained, the crystal structure is displayed so as to include the outer perimeters of the through pores without any omissions and is rotated, and then the presence or absence of the through pores is observed from all directions. FIG. 1 to FIG. 3 each show through pores observed from a predetermined direction by rotating the obtained crystal structure. FIG. 3 shows an inscribed circle 11 of the through pore observed from a direction shown in FIG. 3. The inscribed circles of all the through pores observed from each direction are identified, and a through pore Pₘₐₓ having the largest inscribed circle diameter is identified among all the through pores observed.

(a1) In order to observe all the through pores so as to include the outer perimeters without any omissions, observation of a unit cell is performed first. If the through pore with a missing outer perimeter is present, a 2×2×2 expansion of the unit cell may be displayed and observed.

(a2) Next, the through pore Pₘₐₓ is observed in each of a specific cross section ab, a specific cross section bc, and a specific cross section ca as described below.

The cross section ab is a cut cross section, of the through pore Pₘₐₓ, along a plane parallel to an a-b plane of a unit cell, and is a plane obtained by cutting the through pore Pₘₐₓ where the through pore Pₘₐₓ penetrates. In some cases, a plurality of cross sections that are parallel to the a-b plane of the unit cell and along which the through pore Pₘₐₓ can be cut where the through pore Pₘₐₓ penetrates, are present. If the plurality of cross sections are present, the cross section ab is a cross section in which the overlapping portion with the through pore Pₘₐₓ is the largest.

The cross section bc is a cross section obtained by replacing the cross section ab with the cross section bc and the a-b plane with a b-c plane, in identifying of the cross section ab.

The cross section ca is a cross section obtained by replacing the cross section ab with the cross section ca and the a-b plane with a c-a plane.

An observation region in each of these cross sections ab, bc, ca corresponds to that in the procedure (a1).

After the through pore Pₘₐₓ is observed in each of the cross section ab, the cross section bc, and the cross section ca, the longest length is found among inner diameters of the through pore Pₘₐₓ obtained by cutting the through pore Pₘₐₓ along lines orthogonal to the central axis of the through pore Pₘₐₓ, and the longest one, among the longest length in the cross section ab, the longest length in the cross section bc, and the longest length in the cross section ca, is set as the largest pore diameter L of the through pore Pₘₐₓ.

(a3) Further, in the cross section in which the largest pore diameter L is determined, the smallest length, among inner diameters of the through pore Pₘₐₓ obtained by cutting the through pore Pₘₐₓ along the lines orthogonal to the central axis of the through pore Pₘₐₓ, is set as the smallest pore diameter S.

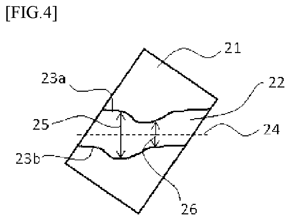

FIG. 4 shows a cross section 21 (one of the cross section ab, the cross section bc, and the cross section ca) in which a largest pore diameter L25 of a through pore Pₘₐₓ 22 is determined. Reference characters 23a, 23b represent the outer perimeter of the through pore Pₘₐₓ 22, and a reference character 24 represents the central axis of the through pore Pₘₐₓ 22. **In** the cross section 21 in which the largest pore diameter L25 is determined, a reference character 26 represents the smallest length among inner diameters of the through pore Pₘₐₓ 22 obtained by cutting the through pore Pₘₐₓ 22 along lines orthogonal to the central axis 24 of the through pore Pₘₐₓ 22, and is the smallest pore diameter S.

It is considered that, since the value of the largest pore diameter L/the smallest pore diameter S, which is determined as described above, being 1.06 or more means that the through pore has a winding shape, the internal surface area of the through pore is increased, and thus, substances such as water, carbon dioxide, and hydrogen are adsorbed easily.

The value of the largest pore diameter L and the value of the smallest pore diameter S are not limited as long as the largest pore diameter L/the smallest pore diameter S is 1.06 or more, and the largest pore diameter L is 2.5 to 20 Å and the smallest pore diameter S is 1.5 to 15 Å, for example.

The metal ion constituting the MOF is an ion of at least one metal selected from the group consisting of Al, Ga, In, Ti, V, Cr, Mn, Fe, Co, Cu, Zr, and Hf, is preferably an ion of at least one metal selected from the group consisting of Al, Ga, In, Ti, V, Co, Zr, and Hf, is more preferably an ion of at least one metal selected from the group consisting of Al, Ga, In, Ti, V, Zr, and Hf, is further preferably an ion of at least one metal selected from the group consisting of Al, In, and Ti, and is particularly preferably an Al ion, or an In ion and/or a Ti ion.

The organic ligand constituting the MOF includes at least one kind among organic ligands each having a group (carboxylate) represented by R(COO⁻)ₙ in which R represents an aromatic hydrocarbon group or pyrrole, and may have a functional group X which is -OH, -NH₂, or -S-S-, and n is 2 or more and 3 or less.

The number of carbon atoms in the aromatic hydrocarbon group is preferably in a range of 6 or more and 30 or less, 6 or more and 24 or less, 6 or more and 18 or less, 6 or more and 12 or less, and 6 or more and 10 or less, in this order. Specific examples include a group that is obtained by removing n (preferably, 2 or 3) hydrogen atoms from benzene or biphenyl and that may have a functional group X (particularly, -OH, -NH₂, or -S-S-).

R(COO⁻)ₙ is a group in which n protons are removed from R(COOH)ₙ.

Examples of the R(COOH)ₙ with n being equal to 2, that is, dicarboxylic acid, include 1,2-benzene dicarboxylic acid (phthalic acid), 1,3-benzene dicarboxylic acid (isophthalic acid), 1,4-benzene dicarboxylic acid (terephthalic acid), 2-aminoterephthalic acid, 2,5-dihydroxy terephthalic acid, 2,2'-dithiodibenzoic acid, perylene-3,9-dicarboxylic acid, 4,4'-dihydroxybiphenyl-3,3'-dicarboxylic acid, 4,4'-diamino-1,1'-biphenyl-3,3'-dicarboxylic acid, 4,4'-diaminobiphenyl-3,3'-dicarboxylic acid, 1,1'-binaphthyl dicarboxylic acid, phenylindandicarboxylic acid, naphthalene-1,8-dicarboxylic acid, 1,4-naphthalenedicarboxylic acid, 2,6-naphthalenedicarboxylic acid, 12,3-naphthalenedicarboxylic acid, anthracene-2,3-dicarboxylic acid, 2',3'-diphenyl-p-terphenyl-4,4''-dicarboxylic acid, 5-tert-butyl-1,3-benzene dicarboxylic acid, 5-hydroxy-1,3-benzene dicarboxylic acid, 2,5-dihydroxy-1,4-benzene dicarboxylic acid, 4,4'-dihydroxy-diphenylmethane-3,3'-dicarboxylic acid, 1H-pyrrole-2,5-dicarboxylic acid, 1-methylpyrrole-3,4-dicarboxylic acid, and 1-benzyl-1H-pyrrole-3,4-dicarboxylic acid. The dicarboxylic acid is preferably at least one selected from the group consisting of isophthalic acid, 1H-pyrrole-2,5-dicarboxylic acid, terephthalic acid, 2-aminoterephthalic acid, 2,2'-dithiodibenzoic acid, and 4,4'-dihydroxybiphenyl-3,3'-dicarboxylic acid.

Examples of the R(COOH)ₙ with n being equal to 3, that is, tricarboxylic acid, include trimellitic acid, trimesic acid (benzene tricarboxylic acid), biphenyl-3,4',5-tricarboxylic acid, and 1,3,5-tris(4-carboxyphenyl)benzene. Trimellitic acid, benzene tricarboxylic acid, or 1,3,5-tris(4-carboxyphenyl)benzene is preferable, and benzene tricarboxylic acid is particularly preferable.

The R is preferably any one of the following (A-1) to (A-10).

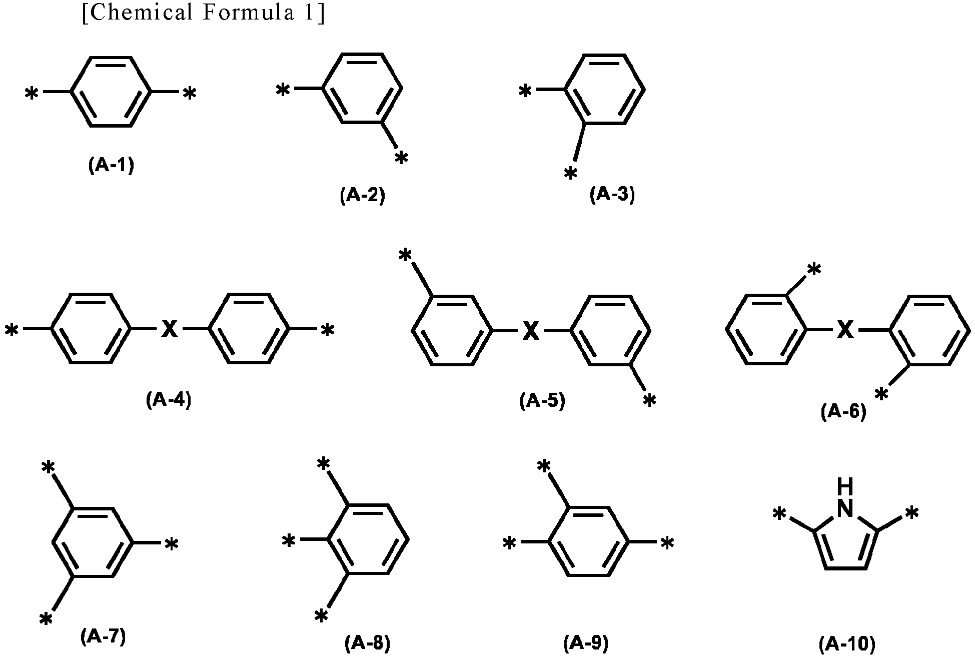

In the (A-1) to (A-10), at least one hydrogen atom bonded to a carbon atom may be substituted by -OH or -NH₂, and -X- represents -S-S- or a single bond.

As the organic ligand constituting the MOF of the present invention, a different kind of an organic ligand other than carboxylates represented by the R(COO⁻)ₙ may be further included, and examples of the organic ligand include at least one selected from the group consisting of urea, pyrazine, oxazole, isoxazole, thiazole, imidazole, pyrazole, 1,2,3-thiadiazole, pyridazine, pyrimidine, purine, pteridine, 2,2'-bipyridine, and 4,4'-bipyridine, and 4,4'-bipyridine is particularly preferable.

The molar ratio (metal ion/organic ligand) of the metal ion to the organic ligand (in a case of a plurality of kinds of organic ligands, the total amount thereof) is preferably 0.1 or more, more preferably 0.3 or more, further preferably 0.5 or more, and particularly preferably 0.9 or more, and is preferably 5 or less, more preferably 4 or less, further preferably 3 or less, and particularly preferably 2.5 or less. The molar ratio is particularly preferably 0.9 or more and 4 or less, and most preferably 0.9 or more and 2.5 or less.

Next, a method for producing the MOF of the present invention will be described. The MOF of the present invention can be obtained by reacting, in a solvent, a metal compound containing the metal ion constituting the MOF and one or more kinds of organic compounds for the organic ligands constituting the MOF. **In** addition, an inorganic compound for an inorganic ligand is also preferably mixed with the solvent together with the metal compound or the organic compounds, as necessary.

Specifically, it is preferable that a solution A obtained by completely dissolving, in a solvent, one of the metal compound containing the metal ion and the organic compounds for the organic ligands is prepared first and the other thereof is added dropwise (at this time, it is also preferable that a solution B obtained by dissolving the other thereof in a solvent is added dropwise). In addition, it is also preferable that a solution X obtained by completely dissolving, in a solvent, both the metal compound containing the metal ion and the organic compounds for the organic ligands is prepared, and then a different kind of a metal compound or a different kind of an organic compound other than the metal compound or the organic compounds in the solution X is added dropwise, or a solution Y obtained by completely dissolving, in a solvent, a different kind of a metal compound or a different kind of an organic compound other than the metal compound or the organic compounds in the solution X is added dropwise. The temperature during dropwise addition is preferably ordinary temperature (specifically, about 20 to 30°C). **In** addition, an amount of the metal compound or the organic compounds to be added dropwise is preferably 1.0 mmol/min. or less. The dropping rate is preferably 0.8 mmol/min. or less, and may be 0.01 mmol/min. or more, preferably 0.1 mmol/min. or more, and more preferably 0.3 mmol/min. or more. In particular, the metal compound, or a solution obtained by dissolving the metal compound in a solvent is preferably added dropwise to a solution obtained by dissolving the organic compounds in a solvent, and, at this time, adding dropwise with the above-described dropping rate is further preferable.

The metal compound that contains the metal ion constituting the MOF is preferably metal sulfate, metal nitrate, metal acetate, metal chloride, metal bromide, or metal alkoxide.

The organic compounds for the organic ligands constituting the MOF preferably include one or more selected from the group consisting of polyvalent carboxylic acids represented by R(COOH)ₙ. R and n are equivalent to R and n described above for the carboxylates represented by R(COO⁻)ₙ, and all the above descriptions about R and n, also including preferable aspects thereof, can be referred to.

The organic compounds for the organic ligands preferably include dicarboxylic acid or tricarboxylic acid. For specific examples of the dicarboxylic acid or the tricarboxylic acid, the dicarboxylic acid or the tricarboxylic acid described for the carboxylates represented by R(COO⁻)ₙ and preferable ranges thereof can be referred to.

As the organic compounds for the organic ligands, in addition to one or more selected from the group consisting of the polyvalent carboxylic acids represented by R(COOH)ₙ, at least one selected from the group consisting of urea, pyrazine, oxazole, isoxazole, thiazole, imidazole, pyrazole, 1,2,3-thiadiazole, pyridazine, pyrimidine, purine, pteridine, 2,2'-bipyridine, and 4,4'-bipyridine is preferably used. In addition, as the above-described inorganic compound for the inorganic ligand, alkali metal hydroxide or alkali metal azide is preferably used. Furthermore, in some cases, an inorganic ligand such as OH⁻, O²⁻, or OH₂ derived from the solvent for dissolving the metal compound and the organic compounds, water in the air, etc., is included in the MOF.

The solvent for dissolving the metal compound containing the metal ion or the organic compounds for the organic ligands is preferably water, an alcohol-based solvent such as methanol or ethanol, or an amide-based solvent such as dimethylformamide, and only one of these solvents may be used or two or more of these solvents may be mixed and used.

In a case where the solution A, the solution B, or the solution X obtained by completely dissolving the metal compound containing the metal ion is prepared, each ratio of the metal compound to the solvent (metal compound/solvent) in the solution is preferably 0.01 mol/L or more, more preferably 0.1 mol/L or more, and further preferably 0.18 mol/L or more, and is preferably 1 mol/L or less, more preferably 0.7 mol/L or less, further preferably 0.5 mol/L or less, and particularly preferably 0.370 mol/L or less. The ratio is particularly preferably 0.18 mol/L or more and 0.5 mol/L or less, and is most preferably 0.18 mol/L or more and 0.370 mol/L or less.

In a case where the solution A, the solution B, or the solution X obtained by completely dissolving the organic compounds for the organic ligands is prepared, each ratio of the organic compounds to the solvent (organic compounds/solvent) in the solution is preferably 0.01 mol/L or more, more preferably 0.1 mol/L or more, and further preferably 0.2 mol/L or more, and is preferably 1.5 mol/L or less, more preferably 1 mol/L or less, and further preferably 0.7 mol/L or less.

Furthermore, in a case where the inorganic compound for the inorganic ligand is contained in the solution A, the solution B, or the solution X, each ratio of the inorganic compound to the solvent (inorganic compound/solvent) in the solution is preferably 0.05 mol/L or more and is preferably 1 mol/L or less.

The molar ratio between the metal compound containing the metal ion and the organic compounds for the organic ligands may be adjusted such that the ratio of the molar quantity of metal atoms in the metal compound to the molar quantity of the organic compounds is equal to the above-described molar ratio of the metal ion to the organic ligands.

It is important that, after the above-described dropwise addition has finished, the resulting product is at least refluxed, stirred, or left as it is at a temperature of room temperature (e.g., 25°C) to 200°C for about 5 minutes to 100 hours. More specifically, it is important to react the metal compound and the organic compounds for 72 hours or more in a case where the temperature is room temperature to lower than 100°C, for 20 hours or more in a case where the temperature is 100°C or higher and lower than 130°C, and for 15 hours or more in a case where the temperature is 130°C or higher and 200°C or lower. The obtained reaction product is separated from the solvent through centrifugation, filtration, or the like, washed, and dried, whereby a desired MOF can be obtained.

This application claims priority to Japanese Patent Application No. 2022-174075 filed on October 31, 2022 and Japanese Patent Application No. 2022-174076 filed on October 31, 2022, the entire contents of which are incorporated herein by reference.

### EXAMPLES

The present invention will be described in more detail below by means of Examples. The present invention is not limited by the following Examples, and can also be carried out with appropriate modifications being made within the scope of the gist described above and below, and any of these modifications are included in the technical scope of the present invention.

### Example 1

In a 20 mL eggplant flask, 3.233 mmol of isophthalic acid and 2.5 mL of dimethylformamide (DMF) were mixed at 25°C and isophthalic acid was completely dissolved, to obtain a solution A. Separately, 3.693 mmol of Al₂(SO₄)₃·nH₂O (n=14 to 18) and 10 mL of ion-exchanged water were mixed and Al₂(SO₄)₃·nH₂O was completely dissolved to prepare a solution B, and the solution B was added dropwise to the solution A at 25°C over 5 minutes. The ratio of isophthalic acid to the solvent was 1.29 mol/L in the solution A, the ratio of Al₂(SO₄)₃·nH₂O to the solvent was 0.369 mol/L in the solution B, and the dropping rate for Al₂(SO₄)₃·nH₂O was 0.739 mmol/min. Then, the resulting solution was refluxed at 125°C for 24 hours, to obtain a suspension. Decantation was performed on the suspension. The precipitated solid product was subjected to three times of centrifugal washing with 10 ml of water. The obtained filter cake was dried in a vacuum drying oven at 80°C for 24 hours, at 100°C for 24 hours, and at 120°C for 48 hours, to obtain 0.66 g of a substance (yield: 99%).

### Example 2

In a 100 mL eggplant flask, 5.169 mmol of 1H-pyrrole-2,5-dicarboxylic acid, 13.5 mL of ion-exchanged water, and 10.42 mmol of sodium hydroxide were mixed at 25°C, and 1H-pyrrole-2,5-dicarboxylic acid and sodium hydroxide were completely dissolved, to obtain a solution A. Separately, 2.555 mmol of Al₂(SO₄)₃·nH₂O (n=14 to 18) and 13.35 mL of ion-exchanged water were mixed and Al₂(SO₄)₃·nH₂O was completely dissolved to prepare a solution B, and the solution B was added dropwise to the solution A at 25°C over 5 minutes. The ratio of 1H-pyrrole-2,5-dicarboxylic acid to the solvent was 0.383 mol/L in the solution A, the ratio of Al₂(SO₄)₃·nH₂O to the solvent was 0.191 mol/L in the solution B, and the dropping rate for Al₂(SO₄)₃·nH₂O was 0.511 mmol/min. Then, the resulting solution was refluxed at 100°C for 24 hours, to obtain a suspension. Decantation was performed on the suspension. The precipitated solid product was subjected to three times of washing with 20 ml of water and filtering and three times of washing with 20 ml of ethanol and filtering, and the obtained filter cake was dried in a vacuum drying oven at 60°C for 24 hours, to obtain 0.88 g of a substance (yield: 87%).

### Example 3

In a 50 mL pressure-resistant container (including a Teflon (registered trademark) inner cylinder) made of SUS-304, 3.000 mmol of 2-aminoterephthalic acid was completely dissolved in 25 mL of a mixture obtained by mixing dimethylformamide (DMF) and MeOH at a ratio of 1/1 (volume ratio), to obtain a solution A. 1.518 mmol of Ti[OCH(CH₃)₂]₄ was added dropwise to the solution A at 25°C over 20 minutes, and the mixture was stirred by placing a stirrer chip for 5 minutes. The ratio of 2-aminoterephthalic acid to the solvent was 0.12 mol/L in the solution A, and the dropping rate for Ti[OCH(CH₃)₂]₄ was 0.0759 mmol/min. Then, the resulting product was left as it was at 150°C for 16 hours. The obtained precipitated solid product was subjected to three times of washing with 10 mL of DMF and filtering, and the obtained filter cake was dried under reduced pressure in a vacuum drying oven at 120°C for 24 hours and at 150°C for 24 hours, to obtain 0.62 g of a substance (yield: 89.4%).

### Example 4

In a 50 mL vial, 5 ml of DMF was added to 2.496 mmol of terephthalic acid, and the mixture was stirred and terephthalic acid was completely dissolved by placing a stirrer chip, to obtain a solution A. Separately, to 2.654 mmol of indium(III) nitrate hydrate, 5 ml of dimethylformamide (DMF) was added and then 2 ml of ethanol was added. Indium(III) nitrate hydrate was completely dissolved by placing a stirrer chip to obtain a solution B, and the solution B was added dropwise to the solution A over 20 minutes. The resulting solution was stirred for 20 minutes. The ratio of terephthalic acid to the solvent was 0.499 mol/L in the solution A, the ratio of indium(III) nitrate hydrate to the solvent was 0.379 mol/L in the solution B, and the dropping rate for indium(III) nitrate hydrate was 0.133 mmol/min. Then, the resulting product was left as it was at 120°C for 48 hours. The obtained precipitated solid product was subjected to three times of washing with 30 mL of DMF and filtering, and the obtained filter cake was dried under reduced pressure in a vacuum drying oven at 120°C for 24 hours, to obtain 0.49 g of a substance (yield: 89.1%).

### Example 5

In a 1 L eggplant flask, 3.05 mmol of cobalt(II) chloride hexahydrate, 10.42 mmol of 2,2'-dithiodibenzoic acid, 10.40 mmol of sodium azide, and 150 mL of DMF were mixed at 25°C, and cobalt(II) chloride hexahydrate, 2,2'-dithiodibenzoic acid and sodium azide were completely dissolved, to obtain a solution A. Separately, 3.08 mmol of benzene tricarboxylic acid, 3.10 mmol of 4,4'-bipyridine, and 150 ml of ethanol were mixed, and benzene tricarboxylic acid and 4,4'-bipyridine were completely dissolved, to prepare a solution B, and the solution B was added dropwise to the solution A at 25°C over 3 hours. The ratio of cobalt(II) chloride hexahydrate to the solvent was 0.0203 mol/L in the solution A, the ratio of 2,2'-dithiodibenzoic acid to the solvent was 0.0695 mol/L in the solution A, and the ratio of the total of the benzene tricarboxylic acid and 4,4'-bipyridine to the solvent was 0.0412 mol/L in the solution B. In addition, the dropping rate for the total amount of benzene tricarboxylic acid and 4,4'-bipyridine was 0.0343 mmol/min. Then, the resulting solution was stirred at 25°C for four days, to obtain a suspension. The obtained precipitated solid product was subjected to three times of washing with 30 mL of DMF and filtering, and the obtained filter cake was dried under reduced pressure in a vacuum drying oven at 120°C for 24 hours, to obtain 1.16 g of a substance (yield: 90.0%).

### Example 6

In a 100 mL pressure-resistant container (including a Teflon (registered trademark) inner cylinder) made of SUS-304, 7.483 mmol of terephthalic acid was completely dissolved in 25 mL of a mixture obtained by mixing dimethylformamide (DMF) and MeOH at a ratio of 9/1 (volume ratio), to obtain a solution A. The solution A was added dropwise to 4.523 mmol of Ti[OCH(CH₃)₂]₄ at 25°C over 30 minutes and mixed, and the mixture was stirred by placing a stirrer chip for 5 minutes. The ratio of terephthalic acid to the solvent was 0.299 mol/L in the solution A, and the dropping rate for terephthalic acid was 0.249 mmol/min. Then, the resulting product was left as it was at 150°C for 16 hours. The obtained precipitated solid product was subjected to three times of washing with 10 mL of DMF and filtering, and the obtained filter cake was dried under reduced pressure in a vacuum drying oven at 120°C for 24 hours and at 150°C for 24 hours, to obtain 0.35 g of a substance (yield: 24.6%).

### Comparative Example 1

To a 500 mL eggplant flask, formic acid (2.69 mol), acetic anhydride (1.08 mol), and Ti(OCH(CH₃)₂)₄ (0.067 mol) were added. The mixture was stirred at 25°C for 30 minutes, then was heated to 120°C, and was refluxed for 12 hours. The obtained turbid reaction solution was subjected to three times of washing with 100 ml of acetone while being heated to 50°C, to obtain 12.03 g of a white solid A (yield: 74.8%).

In a 500 mL pressure-resistant container (including a Teflon (registered trademark) inner cylinder) made of SUS-304, 0.23 mmol of the white solid A was dissolved in a solution obtained by mixing acetic anhydride (10 mL) and acetic acid (10 mL). 55.2 mmol of 5-aminoisophthalic acid was added to the resulting solution all at once, and then 1 mL of methanol was mixed therein at 25°C. The mixture was stirred by placing a stirrer chip for 5 minutes. Then, the resulting product was left as it was at 180°C for 48 hours. The obtained precipitated solid product was subjected to three times of washing with 30 mL of acetone and filtering, and the obtained filter cake was air-dried for 24 hours, to obtain 0.20 g of a substance (yield: 47.9%).

### Comparative Example 2

In a 50 mL eggplant flask, 2.54 mmol of 2,5-thiophenedicarboxylic acid and 4 mL of DMF were mixed at 25°C and 2,5-thiophenedicarboxylic acid was completely dissolved, to obtain a solution A. Separately, 5.64 mmol of Al₂(SO₄)₃·nH₂O (n=14 to 18) and 16 ml of ion-exchanged water were mixed and Al₂(SO₄)₃·nH₂O was completely dissolved, to prepare a solution B, and the solution B was added to the solution A all at once at 25°C. Then, the resulting solution was refluxed while being stirred at 135°C for 24 hours, to obtain a suspension. The obtained precipitated solid product was subjected to three times of washing with 30 mL of DMF and filtering and three times of washing with 30 mL of methanol and filtering, and the obtained filter cake was dried under reduced pressure in a vacuum drying oven at 80°C for 24 hours, to obtain 0.65 g of a substance (yield: 48.2%).

### Comparative Example 3

In a 100 mL pressure-resistant container (including a Teflon (registered trademark) inner cylinder) made of SUS-304, 5.1 mmol of AlCl₃, 2.089 mmol of terephthalic acid, and 60 ml of DMF were mixed all at once at a time, and AlCl₃ and terephthalic acid were dissolved by applying ultrasonic waves at room temperature for 5 minutes. Then, the resulting product was left as it was at 110°C for 20 hours. The obtained precipitated solid product was subjected to three times of washing with 30 mL of DMF and filtering, and the obtained filter cake was dried under reduced pressure in a vacuum drying oven at 60°C for 30 minutes and at 150°C for 12 hours, to obtain 0.38 g of a substance (yield: 90%).

### Comparative Example 4

In a 50 mL eggplant flask, 5.04 mmol of 2,5-furandicarboxylic acid, 10.59 mmol of NaOH, and 15 mL of ion-exchanged water were mixed, and 2,5-furandicarboxylic acid and NaOH were completely dissolved, to obtain a solution A. A solution B obtained by mixing and completely dissolving 5.04 mmol of AlCl₃·6H₂O and 10 mL of ion-exchanged water was added dropwise to the solution A at 25°C over 30 minutes. The ratio of 2,5-furandicarboxylic acid to the solvent was 0.336 mol/L in the solution A, the ratio of AlCl₃·6H₂O to the solvent was 0.504 mol/L in the solution B, and the dropping rate for AlCl₃·6H₂O was 0.168 mmol/min. Then, the resulting solution was refluxed at 25°C for 18 hours, to obtain a suspension. The obtained precipitated solid product was subjected to three times of centrifugal washing with 50 mL of water, and the obtained cake was dried in a vacuum drying oven at 80°C for 24 hours, to obtain a substance.

The substances obtained in Examples and Comparative Examples were evaluated by the following methods.

### (1) Measurement of porosity

The porosity was obtained as follows. Under the following conditions, each of the substances produced in Examples and Comparative Examples was measured using XRD to obtain a cif, and the cif was loaded into software named Mercury, whereby the porosity was obtained by inputting, a void contact surface having a probe radius of 1.5 Å and an approx. grid spacing of 0.7 Å, to the obtained data.
Device: SmartLab manufactured by Rigaku Corporation
Ray source: Cu
Measurement range: 20=3 to 40°
Step size: 0.01°
Scan speed: 3°/min
Measurement temperature: room temperature (25°C)

### (2) Measurement of largest pore diameter L and smallest pore diameter S

Based on rendering in Mercury which was obtained in the above-described measurement of the porosity, a crystal structure including pore size and pore shape was obtained, and a largest pore diameter L and a smallest pore diameter S were determined according to the above-described procedures (a1) to (a3).

### (3) Measurement of desorption amount

The amount of moisture desorbed from the MOF pre-treated under the following condition was measured by using Simultaneous Thermal Analyzer (TG-DTA: Thermogravimetry-Differential Thermal Analysis) manufactured by Rigaku Corporation.

Pre-treatment condition: each substance was held at 25°C for 12 hours under an air atmosphere in which the humidity was adjusted such that the relative pressure was 0.5.

Desorption amount: after the above-described pre-treatment, the temperature was increased at a temperature increase rate of 5°C/min. while nitrogen was caused to flow, and the substance was held at 50°C for 30 minutes. A weight reduction amount W₂₅₋₅₀ in this range (25 to 50°C) was measured, and a desorption amount (mass%) was obtained by dividing the W₂₅₋₅₀ by the weight of the sample subjected to the pre-treatment.

The results are shown in Table 1. In Comparative Example 1, the pore observed in cross section observation in the procedure (a2) was not a through pore, and thus "closed" was described in the cell for largest pore diameter L/smallest pore diameter S.

**[Table 1]**

| | Metal ion | Organic ligand | Metal ion/ Organic ligand (molar ratio) | Porosity (%) | Largest pore diameter L (Å) | Smallest pore diameter S (Å) | Largest pore diameter L / Smallest pore diameter S | Desorption amount (mass %) |
|---|---|---|---|---|---|---|---|---|
| Example 1 | Al | Isophthalic acid | 2.28 | 30.9 | 9.33 | 3.30 | 2.83 | 21.9 |
| Example 2 | Al | 1H-pyrrole-2,5-dicarboxylic acid | 0.99 | 41.1 | 8.91 | 2.76 | 3.23 | 26.76 |
| Example 3 | Ti | 2-aminoterephthalic acid | 0.51 | 55.3 | 4.54 | 4.09 | 1.11 | 14 |
| Example 4 | In | Terephthalic acid | 1.06 | 56.2 | 4.63 | 3.73 | 1.24 | 19.8 |
| Example 5 | Co | Benzene tricarboxylic acid | 0.18 | 57.8 | 3.70 | 3.16 | 1.17 | 12.42 |
| | | 2,2'-dithiodibenzoic acid | | | | | | |
| | | 4,4'-bipyridine | | | | | | |
| Example 6 | Ti | Terephthalic acid | 0.60 | 63.2 | 4.37 | 4.02 | 1.09 | 12.6 |
| Comparative example 1 | Ti | 5-aminoisophthalic Acetic anhydride | 0.06 | 10.1 | 6.51 | 6.20 | Closed. | 0.98 |
| Comparative example 2 | Al | 2,5-thiophenedicarboxylic | 4.44 | 14.6 | 7.05 | 4.64 | 1.51 | 5.86 |
| Comparative example 3 | Al | Terephthalic acid | 2.44 | 78.6 | 29.82 | 9.55 | 3.12 | 4.74 |
| Comparative example 4 | Al | 2,5-furandicarboxylic acid | 1.00 | Crystalline substance could not be confirmed. | | | | |

In each of Examples 1 to 6, a crystalline MOF having a porosity of 20 to 75% and the largest pore diameter L/the smallest pore diameter S of 1.06 or more was obtained. However, in each of Comparative Examples 1 to 3, a MOF does not satisfy at least one of requirements with respect to the largest pore diameter L/the smallest pore diameter and the porosity. In Comparative Example 4, the reflux condition applied after the solution B was added dropwise to the solution A was not appropriate, so that a crystalline substance could not be confirmed in measurement using XRD.

### INDUSTRIAL APPLICABILITY

The MOF of the present invention can be suitably used to adsorb and remove gas or organic molecules, for example. Examples of the gas include water (water vapor), carbon dioxide, hydrogen, carbon monoxide, oxygen, nitrogen, hydrocarbon having 1 to 4 carbon atoms, noble gas, hydrogen sulfide, ammonia, sulfur oxide, nitrogen oxide, and siloxane. Examples of the organic molecule include hydrocarbon having 5 to 8 carbon atoms, alcohol having 1 to 8 carbon atoms, aldehyde having 1 to 8 carbon atoms, carboxylic acid having 1 to 8 carbon atoms, ketone having 1 to 8 carbon atoms, amine having 1 to 8 carbon atoms, ester having 1 to 8 carbon atoms, and amide having 1 to 8 carbon atoms. The organic molecule may contain an aromatic ring.

### DESCRIPTION OF THE REFERENCE CHARACTERS

- 11: inscribed circle
- 21: cross section
- 22: through pore Pₘₐₓ
- 23a, 23b: outer perimeter of through pore Pₘₐₓ
- 24: central axis of through pore Pₘₐₓ
- 25: largest pore diameter L
- 26: smallest pore diameter S

## Claims

1. A metal organic framework comprising
an organic ligand and a metal ion, wherein
a porosity is 20 to 75%,
a ratio of a largest pore diameter L to a smallest pore diameter S (the largest pore diameter L/the smallest pore diameter S) determined through the following procedures (a1) to (a3) is 1.06 or more,
the organic ligand includes at least one kind among organic ligands each having a group represented by R(COO⁻)ₙ in which R represents an aromatic hydrocarbon group or pyrrole, and may have a functional group X which is -OH, - NH₂, or -S-S-, and n is 2 or more and 3 or less, and
the metal ion is an ion of at least one metal selected from the group consisting of Al, Ga, In, Ti, V, Cr, Mn, Fe, Co, Cu, Zr, and Hf,
(a1) a crystal structure determined through X-ray crystal structure analysis is displayed using a space-filling model, and is displayed so as to include outer perimeters of through pores without any omissions, and presence or absence of the through pores is observed from all directions, to identify a through pore Pₘₐₓ having the largest inscribed circle diameter;
(a2) in each of a cross section ab, a cross section bc, and a cross section ca of the through pore Pₘₐₓ, the longest length is found among inner diameters of the through pore Pₘₐₓ obtained by cutting the through pore Pₘₐₓ along lines orthogonal to a central axis of the through pore Pₘₐₓ, and a longest one, among the longest length in the cross section ab, the longest length in the cross section bc, and the longest length in the cross section ca, is set as the largest pore diameter L of the through pore Pₘₐₓ,
the cross section ab being a cut cross section, of the through pore Pₘₐₓ, along a plane parallel to an a-b plane of a unit cell, and being a plane obtained by cutting the through pore Pₘₐₓ where the through pore Pₘₐₓ penetrates,
if a plurality of the cross sections along which the through pore Pₘₐₓ can be cut where the through pore Pₘₐₓ penetrates are present, the cross section ab being the cross section in which an overlapping portion with the through pore Pₘₐₓ is the largest,
the cross section bc being a cross section obtained by replacing the cross section ab with the cross section bc and the a-b plane with a b-c plane, and the cross section ca being a cross section obtained by replacing the cross section ab with the cross section ca and the a-b plane with a c-a plane, in identifying of the cross section ab; and
(a3) in the cross section in which the largest pore diameter L is determined, a smallest length, among inner diameters of the through pore Pₘₐₓ obtained by cutting the through pore Pₘₐₓ along the lines orthogonal to the central axis of the through pore Pₘₐₓ, is set as the smallest pore diameter S.

2. The metal organic framework according to claim 1, wherein the ratio of the largest pore diameter L to the smallest pore diameter S (the largest pore diameter L/the smallest pore diameter S) is 4.0 or less.
